# EUROPÄISCHE PATENTANMELDUNG

(11) **EP 2 944 259 A1**
(43) Veröffentlichungstag der Anmeldung: **18.11.2015**
(21) Anmeldenummer: 14001711.2
(22) Anmeldetag: 15.05.2014
(51) Int. Cl.: A61B 6/04, A61N 5/10

(54) **Patientenpositioniereinrichtung**

(71) Anmelder: Buck Engineering & Consulting GmbH, 72764 Reutlingen (DE)
(72) Erfinder: Buck, Matthias, 72764 Reutlingen (DE); Gerlich, Martin, 70182 Stuttgart (DE); Bouraoui, Mohamed Wassim, 70599 Stuttgart (DE)
(74) Vertreter: Reinhardt, Annette

(57) **Zusammenfassung**

Eine Patientenpositioniereinrichtung (1) umfasst eine Bewegungseinrichtung zur Bewegung einer Patientenaufnahme, die Translationsbewegungen der Patientenaufnahme in alle drei Raumrichtungen und Rotationsbewegungen um alle drei Raumachsen (x, y, z) ermöglicht. Um eine niedrige Aufstiegshöhe zu ermöglichen, ist vorgesehen, dass die Patientenaufnahme mit der Bewegungseinrichtung über eine Schwenkeinrichtung (23) verbunden ist, die eine Schwenkbewegung der Patientenaufnahme gegenüber der Bewegungseinrichtung um eine horizontale Schwenkachse (24) ermöglicht.

## Beschreibung

Die Erfindung betrifft eine Patientenpositioniereinrichtung der im Oberbegriff des Anspruchs 1 angegebenen Gattung.

Aus der US 2005/0234327 A1 ist eine Patientenpositioniereinrichtung bekannt, die einen Roboterarm umfasst, der fünf Rotationsbewegungsachsen aufweist. Der Roboterarm ist an einer Schiene höhenverfahrbar gelagert. Die Patientenliege ist auf dem Handabschnitt des Roboterarms angeordnet, der Schwenkbewegungen der Patientenaufnahme um alle drei Raumachsen ermöglicht.

Der Erfindung liegt die Aufgabe zugrunde, eine Patientenpositioniereinrichtung der gattungsgemäßen Art zu schaffen, die einen vielfältigen Einsatz ermöglicht.

Diese Aufgabe wird durch eine Patientenpositioniereinrichtung mit den Merkmalen des Anspruchs 1 gelöst.

Es ist vorgesehen, dass die Patientenaufnahme mit der Bewegungseinrichtung über eine Schwenkeinrichtung verbunden ist, die eine Schwenkbewegung der Patientenaufnahme gegenüber der Bewegungseinrichtung um eine horizontale Schwenkachse ermöglicht. Die zusätzliche horizontale Schwenkachse der Schwenkeinrichtung erlaubt ein Verschwenken der Bewegungseinrichtung gegenüber der Patientenaufnahme, wobei die Ausrichtung der Patientenaufnahme, beispielsweise eine horizontale Ausrichtung, erhalten bleiben kann.

Dadurch kann die relative Lage der Anbindung der Patientenaufnahme an der Bewegungseinrichtung geändert werden, so dass ein vielfältiger Einsatz möglich ist.

Die Schwenkachse der Schwenkeinrichtung verläuft vorteilhaft parallel zu einer Längsmittelachse der Patientenaufnahme. Die Längsmittelachse ist dabei die Achse, die in Längsrichtung der Patientenaufnahme, bei einer Patientenliege also beispielsweise in Längsrichtung der Patientenliege, verläuft. Dadurch, dass die Schwenkachse parallel zur Längsmittelachse verläuft, wird für die Verschwenkung nur wenig Raum benötigt, da die Patientenaufnahme um ihre Breitseite und nicht um ihre Längsseite verschwenkt. Um ein seitliches Wegschwenken der Bewegungseinrichtung zu ermöglichen, ist vorgesehen, dass die Schwenkeinrichtung eine Schwenkbewegung der Patientenaufnahme gegenüber der Bewegungseinrichtung um einen Winkel von mindestens 60° um die Schwenkachse der Schwenkeinrichtung ermöglicht. Vorzugsweise ermöglicht die Schwenkeinrichtung eine Schwenkbewegung um mindestens 90°, so dass die Bewegungseinrichtung vollständig zur Seite geschwenkt werden kann. Besonders vorteilhaft ist ein Schwenkwinkel von näherungsweise 180°.

Über die Schwenkeinrichtung kann vorteilhaft eine niedrige Aufstiegshöhe der Patienten-aufnahme realisiert werden. Hierzu wird die Bewegungseinrichtung vorteilhaft aus dem Bereich unterhalb der Patientenaufnahme weggeschwenkt. Das seitliche Wegschwenken kann dabei über die Bewegungseinrichtung erfolgen. Die Schwenkeinrichtung verschwenkt vorteilhaft die Patientenaufnahme in Gegenrichtung, so dass die Patientenauf-nahme in der gewünschten Ausrichtung, vorzugsweise horizontal, bleibt. Dadurch ist es möglich, eine sehr niedrige Aufstiegsposition zu realisieren. Für die Bestrahlung kann die Bewegungseinrichtung vorteilhaft mindestens teilweise unterhalb der Patientenaufnahme angeordnet werden, damit die Zugänglichkeit zum Patienten nicht eingeschränkt ist.

Vorteilhaft fällt die Schwenkachse der Schwenkeinrichtung mit einer Längsmittelachse der Patientenaufnahme zusammen. Die Patientenaufnahme weist vorteilhaft eine Aufnahmefläche für einen Patienten auf. Die Aufnahmefläche ist dabei die Fläche, die das Gewicht des Patienten unterstützt. Die Aufnahmefläche ist vorzugsweise eine näherungsweise horizontal ausgerichtete, ebene Fläche. Die Aufnahmefläche kann jedoch auch zur besseren Lagerung des Patienten eine unebene Fläche sein, die beispielsweise an die Kontur des Patienten angepasst sein kann. Bei einer uneben ausgebildeten Aufnahmefläche ist die Aufnahmefläche dann horizontal angeordnet, wenn ein auf der Aufnahmefläche angeordneter Patient horizontal liegt. Die Unterseite der Patientenaufnahme ist die Seite, die bei horizontal angeordneter Aufnahmefläche dem Raumboden zugewandt liegt. Um die Bewegungseinrichtung in einem kleinen Bauraum seitlich wegschwenken zu können, ist vorteilhaft vorgesehen, dass der Abstand der Schwenkachse der Schwenkeinrichtung zum Raumboden mindestens dem Abstand der Unterseite der Patientenaufnahme zum Raumboden entspricht. Die Schwenkachse kann dabei bei horizontal angeordneter Aufnahmefläche einen horizontalen Abstand und/oder einen vertikalen Abstand zur Längsmittelachse der Patientenaufnahme besitzen. Ist ein vertikaler Abstand vorgesehen, so liegt die Schwenkachse der Schwenkeinrichtung vorteilhaft oberhalb der Längsmittelachse, also in größerem Abstand zum Raumboden als die Längsmittelachse der Patientenaufnahme.

Vorteilhaft ist die Bewegungseinrichtung ein Roboterarm. Der Roboterarm weist vorzugsweise mindestens sechs Rotationsbewegungsachsen auf. Der Roboterarm ermöglicht vorteilhaft Translationsbewegungen in alle Raumrichtungen und Rotationsbewegungen um alle Raumachsen. Der Roboterarm weist vorteilhaft einen Handabschnitt auf, der Bewegungen der Patientenaufnahme um alle drei Raumachsen ermöglicht. Die Schwenkeinrichtung ist vorteilhaft an dem Handabschnitt des Roboterarms festgelegt. Die Schwenkeinrichtung ist demnach zwischen dem Handabschnitt des Roboterarms und der Patientenaufnahme angeordnet. Dadurch kann der Roboterarm von der Patientenaufnahme seitlich weggeschwenkt werden, und die Schwenkeinrichtung kann eine Drehbewegung in Gegenrichtung ausführen, um die Patientenaufnahme in ihrer Ausrichtung, vorzugsweise etwa horizontal, zu halten.

Es ist vorgesehen, dass die Patientenpositioniereinrichtung eine Bestrahlungsposition auf-weist, in der der Handabschnitt zum Raumboden einen Abstand besitzt, der kleiner als ein Abstand der Unterseite der Patientenaufnahme zum Raumboden ist. Der Handabschnitt ist demnach mindestens teilweise unter der Patientenaufnahme angeordnet. Vorzugsweise ist der gesamte Handabschnitt unterhalb der Aufnahmefläche, insbesondere unterhalb der Patientenaufnahme angeordnet. Dadurch ist der Handabschnitt bei einer Bestrahlung des Patienten nicht im Weg. Gleichzeitig wird eine geringere Strahlenbelastung des Handabschnitts erreicht. Die Patientenpositioniereinrichtung besitzt vorteilhaft eine Aufstiegsposition, in der der Abstand des Handabschnitts zum Boden größer ist als der Abstand der Unterseite der Patientenaufnahme zum Raumboden. Die relative Lage des Handabschnitts zur Patientenaufnahme ändert sich demnach zwischen der Bestrahlungsposition und der Aufstiegsposition. Vorzugsweise ist der Handabschnitt in Aufstiegsposition seitlich neben der Patientenaufnahme oder oberhalb der Patientenaufnahme angeordnet. Dadurch kann die Patientenaufnahme sehr weit zum Raumboden abgesenkt werden, und ein Patient kann einfach auf die Patientenaufnahme aufsteigen.

Der Roboterarm besitzt vorteilhaft ein Grundgestell, an dem ein Karussell um eine erste, insbesondere vertikal angeordnete Rotationsbewegungsachse schwenkbar gelagert ist. Der Roboterarm besitzt eine Schwinge, die gegenüber dem Karussell um eine zweite, vorzugsweise horizontal angeordnete Rotationsbewegungsachse schwenkbar gelagert ist. Vorteilhaft weist der Roboterarm einen Arm auf, der gegenüber der Schwinge um eine dritte, insbesondere horizontal angeordnete Rotationsbewegungsachse schwenkbar gelagert ist. Die erste, zweite und dritte Rotationsbewegungsachse können jedoch auch geneigt zur horizontalen und zur vertikalen Richtung im Raum angeordnet sein. Um eine möglichst niedrige Aufstiegshöhe der Patientenaufnahme in Aufstiegsposition zu ermöglichen, ist vorteilhaft vorgesehen, dass der Arm an der Schwinge über einen Zwischenabschnitt festgelegt ist, wobei der Zwischenabschnitt an der Schwinge um eine dritte Rotationsbewegungsachse schwenkbar gelagert ist, und wobei die Rotationsbewegung des Zwischenabschnitts um die dritte Rotationsbewegungsachse an die Bewegung der Schwinge um die zweite Rotationsbewegungsachse über eine Koppeleinrichtung gekoppelt ist. Die zweite Rotationsbewegungsachse und die dritte Rotationsbewegungsachse sind dabei parallel zueinander ausgerichtet. Der Aufbau des Roboterarms mit einem Zwischenabschnitt zwischen Arm und Schwinge stellt einen eigenständigen erfinderischen Gedanken dar, der unabhängig von der Schwenkeinrichtung, die eine Schwenkbewegung der Patientenaufnahme gegenüber der Bewegungseinrichtung um eine horizontale Schwenkachse ermöglicht, ist.

Der Arm ist gegenüber der Schwinge insbesondere um eine vierte, vertikal angeordnete Rotationsbewegungsachse schwenkbar gelagert. Dadurch bleibt die Ausrichtung des Arms bei einer horizontalen Verfahrbewegung in einer Ebene erhalten. Vorteilhaft ist der Arm im Wesentlichen horizontal ausgerichtet. Dadurch kann der Arm in einer horizontalen Ebene in geringem Abstand zum Boden bewegt werden, und die Lage der Aufstiegsposition kann flexibel gewählt werden. Dadurch, dass die Rotationsbewegung des Zwischenabschnitts um die dritte Rotationsbewegungsachse an die Bewegung der Schwinge um die zweite Rotationsbewegungsachse gekoppelt ist, kann ein zusätzlicher Antrieb für die Bewegung des Zwischenabschnitts entfallen. Die Koppeleinrichtung kann dabei eine mechanische Koppeleinrichtung sein. Vorzugsweise umfasst die Koppeleinrichtung ein Koppelelement, das in einem Abstand zur zweiten Rotationsbewegungsachse um eine erste Lagerachse schwenkbar an dem Karussell gehalten ist und das in einem Abstand zur dritten Rotationsbewegungsachse um eine zweite Lagerachse schwenkbar an dem Zwischenelement gehalten ist. Bei einer Verschwenkung der Schwinge gegenüber dem Karussell ändert sich die Lage der ersten Lagerachse relativ zur Schwinge. Dadurch wird der Zwischenabschnitt über das Koppelelement um die dritte Rotationsbewegungsachse verschwenkt. Der Abstand der Lagerachsen zu den zugeordneten Rotationsbewegungsachsen ist dabei vorteilhaft gleich, so dass der Verschwenkwinkel des Zwischenabschnitts dem Verschwenkwinkel der Schwinge entspricht und die Ausrichtung des Arms sich nicht ändert.

Um eine möglichst geringe Aufstiegsposition zu ermöglichen, ist der Arm vorteilhaft gekröpft ausgebildet. Der Arm besitzt dabei einen ersten Abschnitt, mit dem der Arm an dem Zwischenabschnitt gehalten ist und einen zweiten Abschnitt, an dem der Handabschnitt angeordnet ist. Der Abstand der Oberseite des ersten Abschnitts zum Raumboden ist dabei größer als der Abstand der Oberseite des zweiten Abschnitts zum Raumboden. Der Arm ist demnach an dem der Patientenaufnahme zugewandten Bereich gegenüber dem dem Zwischenabschnitt bzw. der Schwinge zugewandten Bereich zum Boden hin versetzt. Der Versatz ist dabei vorzugsweise so groß gewählt, dass die Patientenaufnahme in Bestrahlungsposition über den Arm gedreht werden kann. Dadurch kann auf einfache Weise eine vergleichsweise freie Positionierung der Patientenaufnahme im Raum erreicht werden.

Ausführungsbeispiele der Erfindung werden im Folgenden anhand der Zeichnung erläutert. Es zeigen:
- Fig. 1: eine perspektivische Darstellung eines ersten Ausführungsbeispiels einer Patientenpositioniereinrichtung in Bestrahlungsposition,
- Fig. 2: eine Seitenansicht auf die Patientenpositioniereinrichtung aus Fig. 1,
- Fig. 3: eine perspektivische Darstellung der Patientenpositioniereinrichtung aus Fig. 1 beim Verschwenken in die Aufstiegsposition,
- Fig. 4: eine Seitenansicht auf die Patientenpositioniereinrichtung in der in Fig. 3 gezeigten Stellung,
- Fig. 5: die Patientenpositioniereinrichtung beim weiteren Verschwenken in perspektivischer Darstellung,
- Fig. 6: die Patientenpositioniereinrichtung in Seitenansicht in der in Fig. 5 gezeigten Position,
- Fig. 7: eine perspektivische Darstellung der Patientenpositioniereinrichtung,
- Fig. 8: eine Seitenansicht auf die Patientenpositioniereinrichtung aus Fig. 7,
- Fig. 9: eine perspektivische Darstellung der Patientenpositioniereinrichtung in Aufstiegsposition,
- Fig. 10: eine Seitenansicht der Patientenpositioniereinrichtung in der Aufstiegsposition,
- Fig. 11 und 12: perspektivische Darstellungen der Koppeleinrichtung in unterschiedlichen Stellungen,
- Fig. 13: eine perspektivische Darstellung eines Ausführungsbeispiels einer Patientenpositioniereinrichtung in Bestrahlungsposition,
- Fig. 14: die Patientenpositioniereinrichtung aus Fig. 13 in Seitenansicht,
- Fig. 15: eine perspektivische Darstellung der Patientenpositioniereinrichtung aus Fig. 13 beim Verschwenken in die Aufstiegsposition,
- Fig. 16: die Patientenpositioniereinrichtung aus Fig. 15 in Seitenansicht,
- Fig. 17: eine Seitenansicht der Patientenpositioniereinrichtung aus den Figuren 13 bis 16 in Aufstiegsposition,
- Fig. 18: eine perspektivische Darstellung eines weiteren Ausführungsbeispiels einer Patientenpositioniereinrichtung,
- Fig. 19: die Patientenpositioniereinrichtung aus Fig. 18 in Seitenansicht,
- Fig. 20: die Patientenpositioniereinrichtung aus Fig. 18 in Aufstiegsposition in perspektivischer Darstellung,
- Fig. 21: die Patientenpositioniereinrichtung in der Position aus Fig. 20 in Seitenansicht,
- Fig. 22: eine perspektivische Darstellung eines Ausführungsbeispiels einer Patientenpositioniereinrichtung in Bestrahlungsposition,
- Fig. 23: die Patientenpositioniereinrichtung aus Fig. 21 in Aufstiegsposition in perspektivischer Darstellung,
- Fig. 24: eine perspektivische Darstellung der Patientenpositioniereinrichtung in einer weiteren Bestrahlungsposition,
- Fig. 25: eine ausschnittsweise perspektivische Darstellung der Patientenpositioniereinrichtung aus den Figuren 22 bis 24.

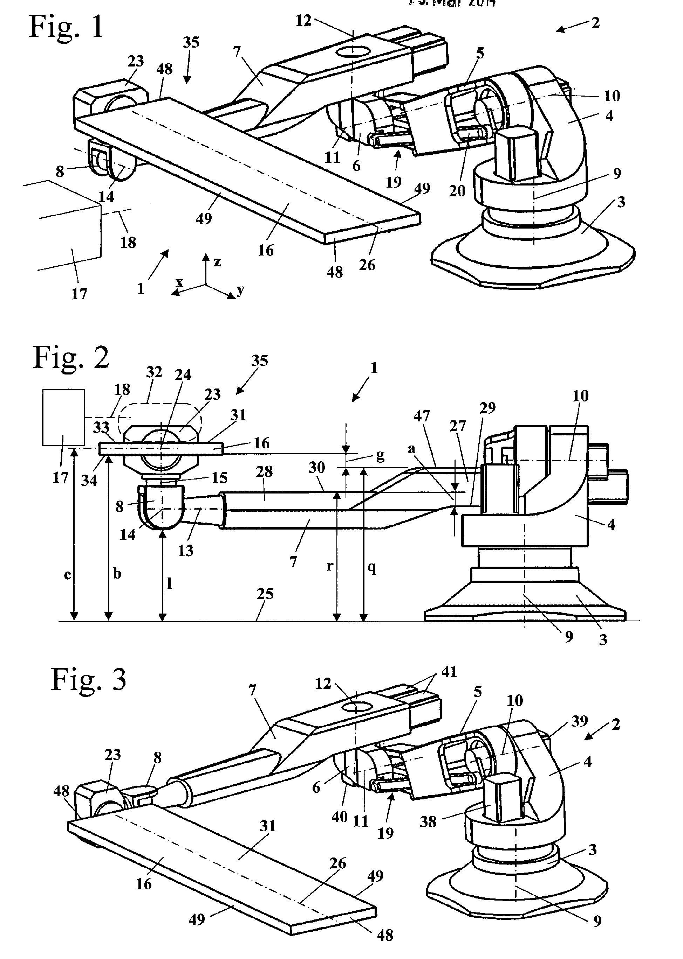

Fig. 1 zeigt eine Patientenpositioniereinrichtung 1. Die Patientenpositioniereinrichtung 1 dient zur Positionierung eines Patienten an einer in Fig. 1 schematisch gezeigten Bestrahlungseinrichtung 17, die einen schematisch dargestellten Strahl 18 zur Bestrahlung eines Patienten erzeugt. Die Bestrahlungseinrichtung 17 kann beispielsweise eine Strahlenquelle für Protonen, Neutronen oder Ionen umfassen. Die Bestrahlungseinrichtung 17 kann auch einen Photonenstrahl, wie beispielsweise einen Röntgen- oder Gammastrahl erzeugen. Auch eine andere Art von Strahlung kann vorteilhaft sein. Die Patientenpositioniereinrichtung 1 kann zusätzlich oder alternativ auch zur Positionierung eines Patienten an einem Bildgebungssystem wie beispielsweise einem Röntgensystem, einem MRT oder dgl. dienen.

Die Patientenpositioniereinrichtung 1 umfasst eine Patientenliege 16, die an einer Bewegungseinrichtung gehalten ist. Im Ausführungsbeispiel ist die Bewegungseinrichtung ein Roboterarm 2. Auch eine andere Bewegungseinrichtung, beispielweise eine Bewegungseinrichtung mit mehreren Translationsbewegungsachsen, kann vorteilhaft sein. Anstatt der Patientenliege 16 kann auch eine andere Patientenaufnahme wie beispielsweise ein Stuhl oder dgl. vorgesehen sein. Der Roboterarm 2 umfasst ein Grundgestell 3, an dem ein Karussell 4 um eine erste, vertikal ausgerichtete Rotationsbewegungsachse 9 drehbar gelagert ist. Am Karussell 4 ist eine Schwinge 5 um eine zweite Rotationsbewegungsachse 10 schwenkbar gelagert. Die zweite Rotationsbewegungsachse 10 ist horizontal angeordnet. Der Roboterarm 2 besitzt einen Arm 7, an dem ein Handabschnitt 8 angeordnet ist. Zwischen der Schwinge 5 und dem Arm 7 ist ein Zwischenabschnitt 6 angeordnet. Der Zwischenabschnitt 6 ist gegenüber der Schwinge 5 um eine dritte, horizontal angeordnete Rotationsbewegungsachse 11 schwenkbar gelagert. Die Rotationsbewegungsachsen 10 und 11 verlaufen parallel zueinander.

Die erste Rotationsbewegungsachse 9 kann auch zur vertikalen Richtung geneigt sein. Die zweite Rotationsbewegungsachse 10 und die dritte Rotationsbewegungsachse 11 können auch zur horizontalen Richtung geneigt sein. Die erste Rotationsbewegungsachse 9 schließt mit der zweiten und der dritten Rotationsbewegungsachse 10, 11 einen Winkel von mehr als 0°, insbesondere von 90° ein, um Bewegungen in alle drei Raumrichtungen zu ermöglichen.

Im Ausführungsbeispiel wird der Zwischenabschnitt 6 um den gleichen Schwenkwinkel wie die Schwinge 5, aber in Gegenrichtung zur Schwinge 5, verschwenkt. Zur Übertragung der Schwenkbewegung ist eine Koppeleinrichtung 19 vorgesehen, die eine Koppelstange 20 umfasst. Wird die Schwinge 5 nach oben verschwenkt, so wird der Zwischenabschnitt 6 um den gleichen Schwenkwinkel in entgegengesetzter Richtung nach unten verschwenkt, so dass die Ausrichtung des Zwischenabschnitts 6 erhalten bleibt. Am Zwischenabschnitt 6 ist der Arm 7 um eine vierte Rotationsbewegungsachse 12 schwenkbar gelagert. Die vierte Rotationsbewegungsachse 12 ist vertikal ausgerichtet. Aufgrund der Kopplung der Schwenkbewegungen von Schwinge 5 und Zwischenabschnitt 6 bleibt die vertikale Ausrichtung der vierten Rotationsbewegungsachse 12 bei Schwenkbewegungen der Schwinge 5 um die zweite Rotationsbewegungsachse 10 erhalten.

Die Patientenliege 16 ist am Handabschnitt 8 über eine Schwenkeinrichtung 23 schwenkbar gelagert. Wie Fig. 1 auch zeigt, besitzt die Patientenliege 16 eine etwa rechteckige Form. Die Patientenliege 16 besitzt zwei einander gegenüberliegende Breitseiten 48 und zwei einander gegenüberliegende Längsseiten 49, wie auch Fig. 3 zeigt. Die Längsseiten 49 sind dabei länger als die Breitseiten 48. Die Schwenkeinrichtung 23 ist an einer der Breitseiten 48 der Patientenliege 16 festgelegt. Die Patientenliege 16 besitzt eine Längsmittelachse 26, die parallel zu den Längsseiten 49 und mittig zwischen den Längsseiten 49 verläuft. In Fig. 1 ist auch eine Rotationsbewegungsachse 14 des Handabschnitts 8 eingezeichnet.

Fig. 2 zeigt die Gestaltung des Handabschnitts 8 im Einzelnen. Der Handabschnitt 8 ist gegenüber dem Arm 7 um eine fünfte Rotationsbewegungsachse 13 des Roboterarms drehbar gelagert. Die fünfte Rotationsbewegungsasche 13 verläuft in Längsrichtung des Arms 7 und ist aufgrund der horizontalen Ausrichtung des Arms 7 ebenfalls horizontal ausgerichtet. Der Handabschnitt 8 besitzt eine sechste Rotationsbewegungsachse 14, die senkrecht zur fünften Rotationsbewegungsachse 13 ausgerichtet ist, sowie eine siebte Rotationsbewegungsachse 15, die senkrecht zur sechsten Rotationsbewegungsachse 14 steht. In der in Fig. 2 gezeigten Anordnung sind die fünfte Rotationsbewegungsachse 13 und die siebte Rotationsbewegungsachse 15 etwa senkrecht zueinander ausgerichtet. Der Handabschnitt ragt etwa senkrecht nach oben.

Aufgrund der Rotationsbewegungsachsen 9 bis 15 ermöglicht der Roboterarm 2 Bewegungen der Patientenliege 16 in allen drei Raumrichtungen, also in Richtung der Raumachsen x, y, z, und um alle drei Raumachsen x, y, z.

In Fig. 2 ist schematisch ein Patient 32 auf der Patientenliege 16 eingezeichnet. Der Patient 32 liegt dabei auf einer Aufnahmefläche 31 der Patientenliege 16, die durch die Oberseite 33 der Patientenliege 16 gebildet ist. In Fig. 2 ist die Patientenliege 16 mit ihrer Aufnahmefläche 31 horizontal angeordnet. Die Aufnahmefläche 31 ist im Ausführungsbeispiel eben ausgebildet. Auch eine gewölbte oder unregelmäßig geformte Aufnahmefläche 31 kann jedoch vorteilhaft sein. Die Fig. 1 und 2 zeigen die Patientenpositioniereinrichtung 1 in einer Bestrahlungsposition 35. Die Patientenliege 16 besitzt eine dem Raumboden 25 zugewandte Unterseite 34, die in der Bestrahlungsposition 35 zum Raumboden 25 einen Abstand b besitzt. In Bestrahlungsposition 35 ist der Handabschnitt 8 unterhalb der Ebene der Aufnahmefläche 31 und auch unterhalb der Ebene der Unterseite 34 der Patientenliege 16 angeordnet. Der Handabschnitt 8 besitzt zum Raumboden 25 einen Abstand 1, der deutlich kleiner als der Abstand b der Unterseite 34 der Patientenliege 16 zum Raumboden 25 ist. Die Schwenkeinrichtung 23 erlaubt eine Verschwenkung der Patientenliege 16 gegenüber dem Handabschnitt 8 um eine Schwenkachse 24. Bei der Patientenpositioniereinrichtung 1 fällt die Schwenkachse 24 mit der Längsmittelachse 26 der Patientenliege 16 zusammen. Die Schwenkachse 24 besitzt in der in Fig. 2 gezeigten Bestrahlungsposition 35, in der die Aufnahmefläche 31 horizontal ausgerichtet ist, zum Raumboden 25 einen Abstand c, der etwas größer als der Abstand b der Unterseite 34 der Patientenliege 16 zum Raumboden 25 ist.

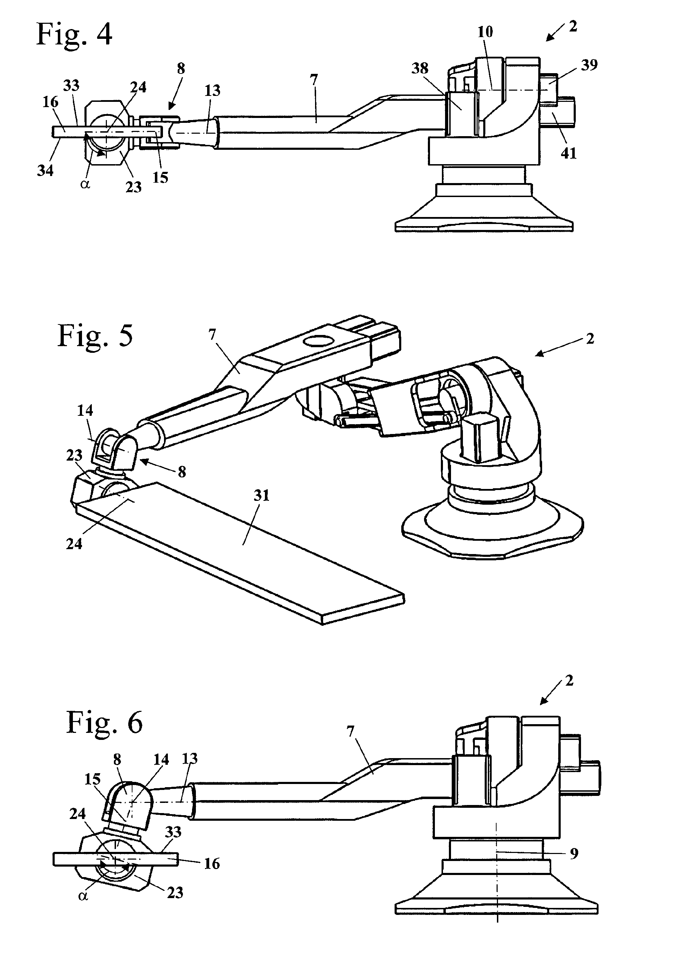

In Fig. 2 ist auch die Gestaltung des Arms 7 im Einzelnen gezeigt. Der Arm 7 ist gekröpft ausgebildet. Der Arm 7 besitzt einen ersten Abschnitt 27, der am Zwischenabschnitt 6 (Fig. 1) angeordnet ist, sowie einen zweiten Abschnitt 28, an dem der Handabschnitt 8 gehalten ist. Der zweite Abschnitt 28 ist gegenüber dem ersten Abschnitt 27 in Richtung auf den Raumboden 25 versetzt. Der erste Abschnitt 27 besitzt eine Oberseite 47, die dem Raumboden 25 abgewandt liegt und einen Abstand q zum Raumboden 25 besitzt. Der zweite Abschnitt 28 besitzt eine Oberseite 30, deren Abstand r zum Raumboden 25 kleiner als der Abstand q ist. Die Abschnitte 27 und 28 sind dabei jeweils etwa horizontal zum Raumboden 25 ausgerichtet. Wie Fig. 2 zeigt, ist der Arm 7 um weniger als die senkrecht gemessene Höhe des ersten Abschnitts 27 abgekröpft. Die Oberseite 30 des zweiten Abschnitts 28 liegt oberhalb einer Unterseite 29 des ersten Abschnitts 27. Wie Fig. 2 zeigt, besitzt die Oberseite 30 zur Unterseite 29 einen Abstand a. Der Arm 7 ist so ausgebildet, dass die Patientenliege 16 in der in den Figuren 1 und 2 gezeigten Bestrahlungsposition 35 über den Arm 7 schwenken kann. Die Oberseite 47 des ersten Abschnitts 27 ist hierzu in geringerem Abstand zum Raumboden 25 angeordnet als die Unterseite 34. Der Abstand q ist kleiner als der Abstand b. Zwischen der Unterseite 34 und der Oberseite 47 des Arms 7 ist ein Abstand g gebildet, der vertikal gemessen ist und der der Differenz zwischen den Abständen b und q entspricht. Der Abstand g ist dabei vorteilhaft so bemessen, dass die Patientenliege 16 selbst bei einer Verformung der Patientenliege 16, beispielsweise aufgrund des Gewichts des Patienten 32, noch über den Arm 7 verschwenkt werden kann. Die Figuren 3 und 4 zeigen die Patientenpositioniereinrichtung 1 nach dem Verschwenken der Schwenkeinrichtung 23 gegenüber der Patientenliege 16 um einen Schwenkwinkel α von etwa 90°. Damit die horizontale Ausrichtung der Aufnahmefläche 31 erhalten bleibt, hat der Handabschnitt 8 eine entsprechende Schwenkbewegung in Gegenrichtung ausgeführt, wobei im Ausführungsbeispiel eine Verschwenkung um alle drei Rotationsbewegungsachsen 13, 14, 15 des Handabschnitts 8 erfolgt ist. Die Rotationsbewegungsachsen 13 und 15 des Handabschnitts 8 liegen in der in den Figuren 3 und 4 gezeigten Position parallel zueinander. In Fig. 3 sind auch die Antriebe des Roboterarms 2 gezeigt. Der Roboterarm 2 besitzt einen ersten Antrieb 38 zur Verschwenkung des Karussells 4 gegenüber dem Grundgestell 3. Ein Antrieb 39 ist zur Verschwenkung der Schwinge 5 gegenüber dem Karussell 4 vorgesehen. In Fig. 3 ist auch ein dritter Antrieb 40 teilweise erkennbar, der zur Verschwenkung des Arms 7 gegenüber dem Zwischenabschnitt 6 dient. Am Arm 7 sind zwei Antriebe 41 angeordnet, die den Handabschnitt 8 verschwenken. Wie Fig. 3 auch zeigt, ist zur Verschwenkung des Zwischenabschnitts 6 gegenüber der Schwinge 5 kein zusätzlicher Antrieb vorgesehen. Die Verschwenkung des Zwischenabschnitts 6 um die Rotationsbewegungsachse 11 erfolgt über die Koppeleinrichtung 19.

Die Figuren 5 und 6 zeigen den Roboterarm 2 nach weiterer Verschwenkung der Schwenkeinrichtung 23 und des Handabschnitts 8. Gegenüber der in Fig. 2 gezeigten Bestrahlungsposition 35 hat die Schwenkeinrichtung 23 die Patientenliege 16 um einen Schwenkwinkel α von etwas weniger als 180° verschwenkt. Die Rotationsbewegungsachse 15 des Handabschnitts 8 ist schräg angeordnet. Der Handabschnitt 8 hat eine Schwenkbewegung in Gegendrehrichtung ausgeführt, so dass die horizontale Ausrichtung der Patientenliege 16 erhalten geblieben ist. In der in den Figuren 5 und 6 gezeigten Anordnung ist der Handabschnitt 8 bereits vollständig oberhalb der Aufnahmefläche 31 bzw. der Oberseite 33 der Patientenliege 16 angeordnet.

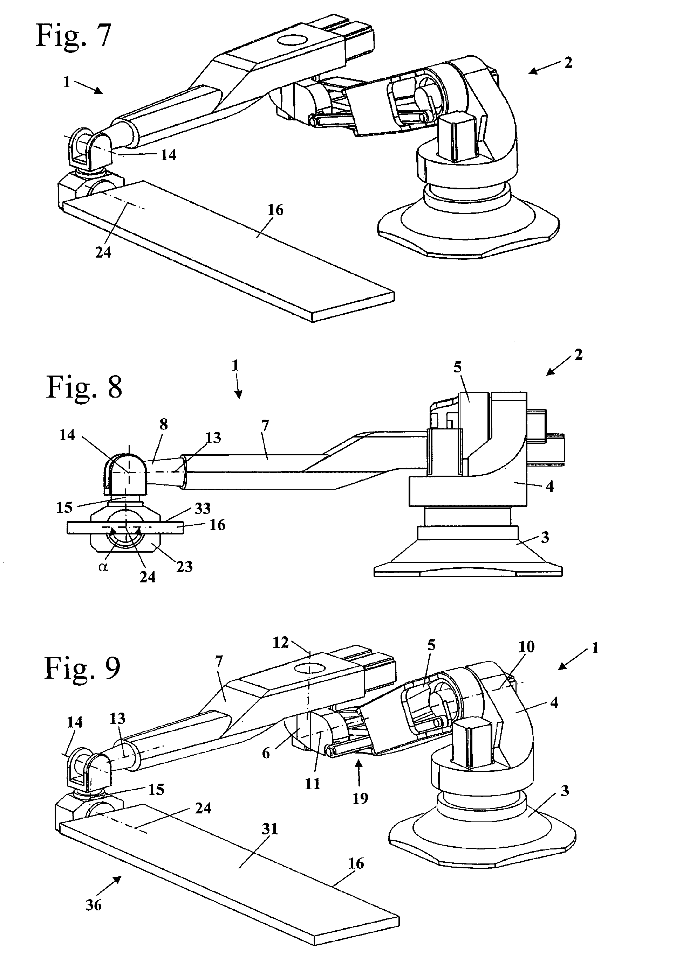

Die Figuren 7 und 8 zeigen die Patientenpositioniereinrichtung 1 nach weiterer Verschwenkung der Patientenliege 16 um die Schwenkachse 24 der Schwenkeinrichtung 23. In der in den Figuren 7 und 8 gezeigten Stellung hat die Schwenkeinrichtung 23 gegenüber der Bestrahlungsposition 35 eine Verschwenkung um einen Schwenkwinkel α von 180° ausgeführt. Die Rotationsbewegungsachse 15 des Handabschnitts 8 ist senkrecht zur Rotationsbewegungsachse 13 und vertikal im Raum ausgerichtet. In den Figuren 1 bis 8 ist die Lage des Arms 7 unverändert.

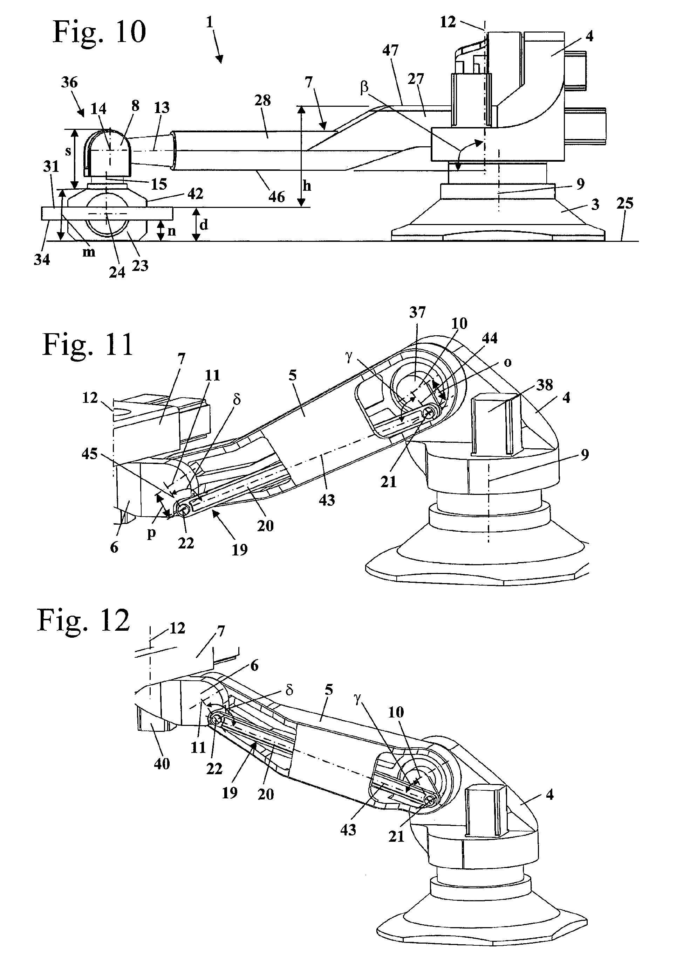

Die Figuren 9 und 10 zeigen die Patientenpositioniereinrichtung 1 in einer Aufstiegsposition 36. In dieser Position besitzt die Aufnahmefläche 31 zum Raumboden 25 einen minimalen Abstand d. Die Schwenkeinrichtung 23 ist bis an den Raumboden 25 oder nahe an den Raumboden 25 abgesenkt. Die Schwenkeinrichtung 23 besitzt ein Gehäuse 42, das in der Aufstiegsposition 36 am Raumboden 25 angeordnet ist. Der Handabschnitt 8 besitzt zum Raumboden 25 einen Abstand m, der deutlich größer als ein Abstand n der Unterseite 34 der Patientenliege 16 zum Raumboden 25 und auch deutlich größer als der Abstand d ist. Die Aufnahmefläche 31 ist in der Aufstiegsposition 36 unterhalb der Oberseite 47 des Arms 7 angeordnet und besitzt zu dieser einen vertikalen Abstand h. Wie Fig. 10 auch zeigt, ist die Unterseite 46 des zweiten Abschnitts 28 des Arms 7 parallel zum Raumboden 25, also horizontal, und senkrecht zur vierten Rotationsbewegungsachse 12 des Arms 7 ausgerichtet. Dadurch kann der Arm 7 sowohl in Bestrahlungsposition 35 (Fig. 2) als auch in Aufstiegsposition 36 (Fig. 10) um die Rotationsbewegungsachsen 12 und 9 verschwenkt und dadurch in einer horizontalen Ebene im Raum bewegt werden. Dadurch, dass die Unterseite 46 parallel zum Raumboden 25 ausgerichtet ist, kann der Abstand zum Raumboden 25 dabei sehr gering sein. In Aufstiegsposition 36 schließt die Unterseite 46 des zweiten Abschnitts 28 des Arms 7 mit der Rotationsbewegungsachse 12 einen Winkel β ein, der etwa 90° beträgt.

Wie Fig. 10 auch zeigt, ist der Abstand d der Aufnahmefläche 31 zum Raumboden 25 in Aufstiegsposition 36 deutlich kleiner als die in vertikaler Richtung gemessene Höhe s des Handabschnitts 8 bei vertikal ausgerichteter Rotationsbewegungsachse 15. Dadurch, dass der Handabschnitt 8 oberhalb der Patientenliege 16 angeordnet ist, kann die Aufnahme-fläche 31 in einem Abstand zum Raumboden 25 angeordnet werden, der kleiner als die Höhe s des Handabschnitts 8 ist.

Die Figuren 11 und 12 zeigen die Gestaltung der Koppeleinrichtung 19 im Einzelnen. Das Karussell 4 besitzt eine Nabe 37, an der eine erste Lagerachse 21 für die Koppelstange 20 ausgebildet ist. Die erste Lagerachse 21 besitzt zur Rotationsbewegungsachse 10 der Schwinge 5 einen Abstand o. In der in Fig. 11 gezeigten Stellung schließen eine Verbindung 44 der Rotationsbewegungsachse 10 mit der ersten Lagerachse 21 und eine Längsmittelachse 43 der Koppelstange 20 einen Winkel γ ein, der im Ausführungsbeispiel etwas kleiner als 90° ist. Die Koppelstange 20 ist mit ihrem zweiten Ende an einer zweiten Lagerachse 22 am Zwischenabschnitt 6 schwenkbar gelagert. Die zweite Lagerachse 22 ist in einem Abstand p zur Rotationsbewegungsachse 11 des Zwischenabschnitts 6 angeordnet. Die Abstände o und p sind vorzugsweise gleich groß. Die Rotationsbewegungsachse 11 ist mit der zweiten Lagerachse 22 über eine Verbindung 45 verbunden, die mit der Längsmittelachse 43 der Koppelstange 20 einen Winkel δ einschließt. Die Verbindungen 44 und 45 bilden mit der Längsmittelachse 43 und einer Verbindungslinie der Rotationsbewegungsachsen 10 und 11 ein Parallelogramm. Die Winkelsumme der Winkel γ und δ beträgt in jeder Stellung 180°. Dadurch bleibt die vertikale Ausrichtung der Rotationsbewegungsachse 12 bei einer Verschwenkung der Schwinge 5 um die zweite Rotationsbewegungsachse 10 erhalten, ohne dass für den Zwischenabschnitt 6 ein zusätzlicher Antrieb benötigt wird. Die gedachten Verbindungen 44 und 45 sind dabei in einer Ebene senkrecht zu den Lagerachsen 21 und 22 eingezeichnet.

In der in Fig. 12 gezeigten Stellung hat sich der Zwischenabschnitt 6 nach oben bewegt. Hierzu wurde die Schwinge 5 um die Rotationsbewegungsachse 10 in den Figuren 11 und 12 im Uhrzeigersinn verschwenkt. Der Winkel γ hat sich verkleinert und der Winkel δ in entsprechendem Maße vergrößert. Die Rotationsbewegungsachse 12 des Arms 7 ist nach wie vor vertikal angeordnet. Die Position der ersten Lagerachse 21 gegenüber dem Karussell ist unverändert. Die Schwinge 5 hat sich gegenüber der ersten Lagerachse 21 bewegt.

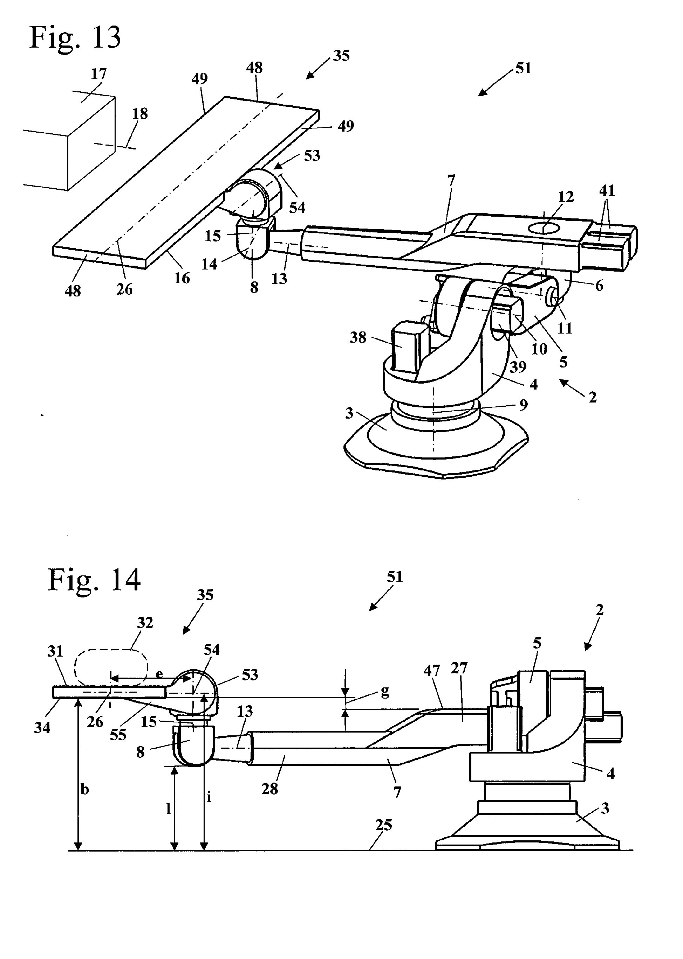

Die Figuren 13 bis 17 zeigen ein Ausführungsbeispiel einer Patientenpositioniereinrichtung 51. Gleiche Bezugszeichen kennzeichnen dabei in allen Figuren einander entsprechende Bauteile, wobei auf die Beschreibung zu den Figuren 1 bis 12 verwiesen wird.

Der Roboterarm 2, der zur Positionierung der Patientenliege 16 der Patientenpositioniereinrichtung 51 dient, entspricht dem Roboterarm 2 des Ausführungsbeispiels aus den Figuren 1 bis 12.

Die Figuren 13 und 14 zeigen die Patientenpositioniereinrichtung 51 in einer Bestrahlungsposition 35. Die Patientenliege 16 ist über eine Schwenkeinrichtung 53 am Handabschnitt 8 des Roboterarms 2 gehalten. Die Schwenkeinrichtung 53 besitzt eine Schwenkachse 54, die parallel zur Längsmittelachse 26 der Patientenliege 16 verläuft. Wie Fig. 14 zeigt, besitzt die Schwenkachse 54 zur Längsmittelachse 26 der Patientenliege 16 einen horizontalen Abstand e, der größer als die halbe Breite der Patientenliege 16 ist. Die Schwenkachse 54 ist demnach neben der Patientenliege 16 angeordnet. Wie Fig. 14 auch zeigt, besitzt die Schwenkeinrichtung 53 einen Stützarm 55, der sich an der Unterseite 34 der Patientenliege 16 erstreckt und zur Versteifung und besseren Fixierung der Patientenliege 16 an der Schwenkeinrichtung 53 dient.

Wie Fig. 14 zeigt, ist der Abstand b der Unterseite 34 der Patientenliege 16 in Bestrahlungsposition 35 deutlich größer als ein Abstand 1 des Handabschnitts 8 zum Raumboden 25. Der Handabschnitt 8 liegt unter der Ebene der Unterseite 34 der Patientenliege 16. Die Schwenkachse 54 besitzt zum Raumboden 25 einen Abstand i, der im Ausführungsbeispiel geringfügig größer als der Abstand b der Unterseite 34 zum Raumboden 25 ist. Die Unterseite 34 verläuft in einer Ebene oberhalb der Oberseite 47 des Arms 7. Zwischen der Oberseite 47 des Arms 7 und der Unterseite 34 der Patientenliege 16 ist ein vertikaler Abstand g gebildet, so dass die Patientenliege 16 auch bei einer Durchbiegung, beispielsweise aufgrund des Gewichts des Patienten, noch über den Arm 7 verschwenkt werden kann.

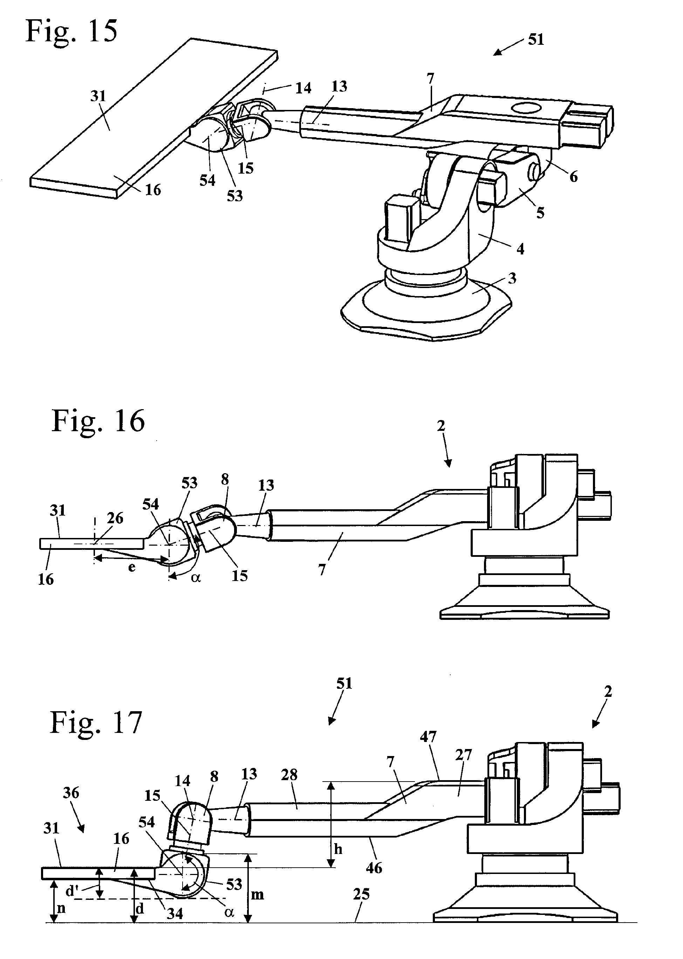

Die Figuren 15 und 16 zeigen die Patientenliege 16 beim Verschwenken in eine Aufstiegsposition 36 (Fig. 17). In der in den Figuren 13 und 14 gezeigten Bestrahlungsposition 35 verläuft die Rotationsbewegungsachse 15 des Handabschnitts 8 senkrecht zur Rotationsbewegungsachse 13 des Handabschnitts 8. Der Handabschnitt 8 ist dabei nach oben ausgerichtet, und die Schwenkeinrichtung 53 ist oberhalb des Handabschnitts 8 angeordnet. In den Figuren 15 und 16 hat die Schwenkeinrichtung 53 die Patientenliege 16 um einen Schwenkwinkel α verschwenkt, der größer als 90° ist. Der Handabschnitt 8 hat eine entsprechende Schwenkbewegung um eine parallel hierzu liegende Raumachse in Gegenrichtung ausgeführt, so dass die horizontale Ausrichtung der Aufnahmefläche 31 erhalten geblieben ist. Die Verschwenkung um eine parallel zur Schwenkachse 54 liegende Achse kann dabei durch eine kombinierte Rotationsbewegung um die Rotationsbewegungsachsen 13, 14 und 15 des Handabschnitts 8 erfolgen. Es kann auch vorgesehen sein, die Rotationsbewegungsachse 14 parallel zur. Schwenkachse 54 auszurichten und anschließend durch Verschwenkung des Handabschnitts 8 um die Rotationsbewegungsachse 14 und Verschwenkung der Patientenliege 16 um die Schwenkachse 54 um den gleichen Winkel in Gegenrichtung eine entsprechende Verstellung zu erreichen.

Fig. 17 zeigt die Patientenpositioniereinrichtung 51 in Aufstiegsposition 36. Die Aufnahmefläche 31 besitzt in Aufstiegsposition 36 einen Abstand d zum Raumboden 25, der vergleichsweise klein ist. Gegenüber der in Fig. 14 gezeigten Bestrahlungsposition 35 hat die Schwenkeinrichtung 53 eine Verschwenkung um einen Schwenkwinkel α, der im Aus-führungsbeispiel etwas kleiner als 180° ist, ausgeführt. Es kann jedoch auch vorgesehen sein, dass die Schwenkeinrichtung 53 eine Verschwenkung um einen Schwenkwinkel α von 180° ausführt. Im Ausführungsbeispiel besitzt die Schwenkeinrichtung 53 zum Raumboden 25 in Aufstiegsposition 36 einen Abstand. Zur Verringerung der Aufstiegshöhe kann der Arm 7 mit dem Handabschnitt 8, der Schwenkeinrichtung 53 und der Patientenliege 16 durch Verschwenkung der Schwinge 5 um die Rotationsbewegungsachse 10 und entsprechende Verschwenkung des Zwischenabschnitts 6 in Gegenrichtung noch abgesenkt werden, bis die Schwenkeinrichtung 53 am Raumboden 25 angeordnet ist, um den Abstand d auf einen in Fig. 17 schematisch eingezeichneten Abstand d' zu verringern. Wie Fig. 17 auch zeigt, besitzt der Handabschnitt 8 einen Abstand m zum Raumboden 25, der größer als der Abstand d ist. Der Handabschnitt 8 ist vollständig oberhalb der Ebene der Aufnahmefläche 31 angeordnet. Die Aufnahmefläche 31 ist unterhalb der Oberseite 47 des Arms 7 angeordnet, und zwar in einem vertikalen Abstand h zur Oberseite 47. Die Aufnahmefläche 31 ist im Ausführungsbeispiel in geringerem Abstand zum Raumboden 25 angeordnet als die Unterseite 46 des zweiten Abschnitts 28 des Arms 7.

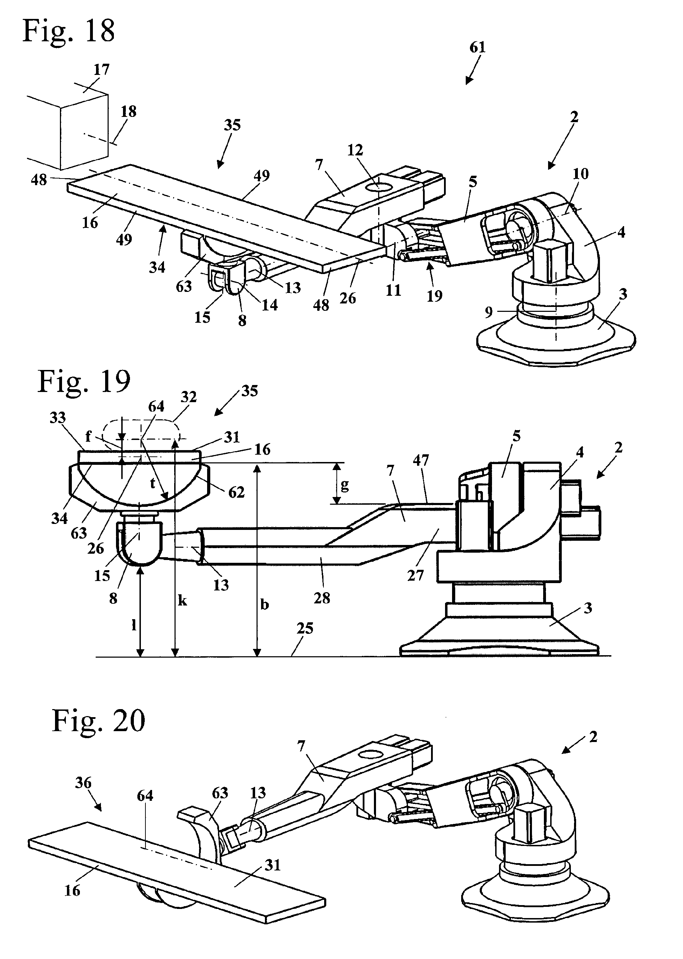

Bei dem in den Figuren 18 bis 21 gezeigten Ausführungsbeispiel einer Patientenpositioniereinrichtung 61 ist zwischen dem Roboterarm 2 und der Patientenliege 16 eine Schwenkeinrichtung 63 vorgesehen, die an der Unterseite 34 der Patientenliege 16 angeordnet ist. Die Schwenkeinrichtung 63 besitzt eine bogenförmige Führung 62 (Fig. 19), die eine Verschwenkung der Patientenliege 16 um eine Schwenkachse 64 erlaubt. Wie Fig. 19 zeigt, besitzt die Schwenkachse 64 in Bestrahlungsposition 35 einen Abstand k zum Raumboden 25, der größer als der Abstand b der Unterseite 34 der Patientenliege 16 zum Raumboden 25 ist. Der Handabschnitt 8 ist in Bestrahlungsposition 35 unter der Schwenkeinrichtung 63 angeordnet. Die Rotationsbewegungsachse 13 des Handabschnitts 8 ist horizontal und die Rotationsbewegungsachse 15 vertikal ausgerichtet. Die Schwenkachse 64 der Schwenkeinrichtung 63 liegt oberhalb der Aufnahmefläche 31. Im Ausführungsbeispiel ist die Schwenkachse 64 senkrecht über der Längsmittelachse 26 der Patientenliege 16 in einem vertikalen Abstand f zur Längsmittelachse 26 und parallel zur Längsmittelachse 26 angeordnet. Wie Fig. 19 auch zeigt, ist ein Radius t der Führung 62 größer als die halbe Breite der Patientenliege 16. Dadurch kann die Schwenkeinrichtung 63 mittig an der Patientenliege 16, also benachbart zur Mitte der Längsseiten 49, angeordnet werden, und die Patientenliege 16 kann durch die Schwenkeinrichtung 63 ragen. Der Handabschnitt 8 ist in Bestrahlungsposition 35 unter der Patientenliege 16 angeordnet. Der Abstand 1 des Handabschnitts 8 zum Raumboden 25 ist deutlich kleiner als der Abstand b der Unterseite 34 der Patientenliege 16 zum Raumboden 25. Auch die Schwenkeinrichtung 63 ist vollständig unterhalb der Patientenliege 16 angeordnet. Die Unterseite 34 der Patientenliege 16 liegt in einer Ebene, die oberhalb der Oberseite 47 des Arms 7 angeordnet ist. Die Patientenliege 16 besitzt dadurch einen vertikalen Abstand g zum Arm 7, so dass die Patientenliege 16 über den Arm 7 verschwenkt werden kann.

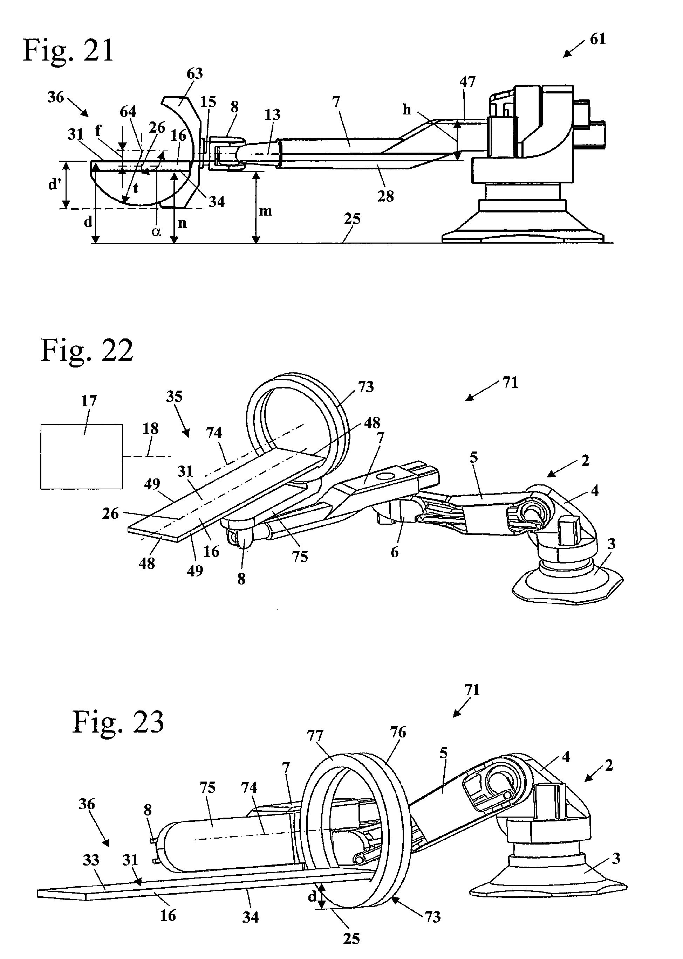

Die Figuren 20 und 21 zeigen die Patientenpositioniereinrichtung 61 in Aufstiegsposition 36. In dieser Position hat die Schwenkeinrichtung 63 die Patientenliege 16 um einen Schwenkwinkel α verschwenkt, der etwa 90° beträgt. Der Handabschnitt 8 hat eine Schwenkbewegung in Gegenrichtung ausgeführt, so dass die horizontale Ausrichtung der Aufnahmefläche 31 der Patientenliege 16 erhalten geblieben ist. Die Unterseite 34 der Patientenliege 16 besitzt zum Raumboden 25 einen Abstand n, der etwas kleiner als ein Abstand m des Handabschnitts 8 zum Raumboden 25 ist. Die Rotationsbewegungsachsen 13 und 15 des Handabschnitts 8 sind parallel zueinander und horizontal ausgerichtet. Der Handabschnitt 8 ist im Bereich neben der Patientenliege 16 angeordnet. Die Aufnahmefläche 31 besitzt zum Raumboden 25 einen Abstand d. Durch Verschwenken der Schwinge 5 kann die Patientenliege 16 noch weiter abgesenkt werden, bis die Aufnahmefläche 31 einen in Fig. 21 schematisch eingezeichneten Abstand d' zum Raumboden 25 aufweist. Der Abstand d' wird dabei durch die Schwenkeinrichtung 63, nämlich den Radius t der Schwenkeinrichtung 63 mit bestimmt. In Aufstiegsposition 36 ist die Aufnahmefläche 31 unterhalb der Oberseite 47 des Arms 7 angeordnet und besitzt zu dieser einen vertikalen Abstand h. Die Patientenliege 16 ist etwa auf der Höhe des Abschnitts 28 des Arms 7 angeordnet.

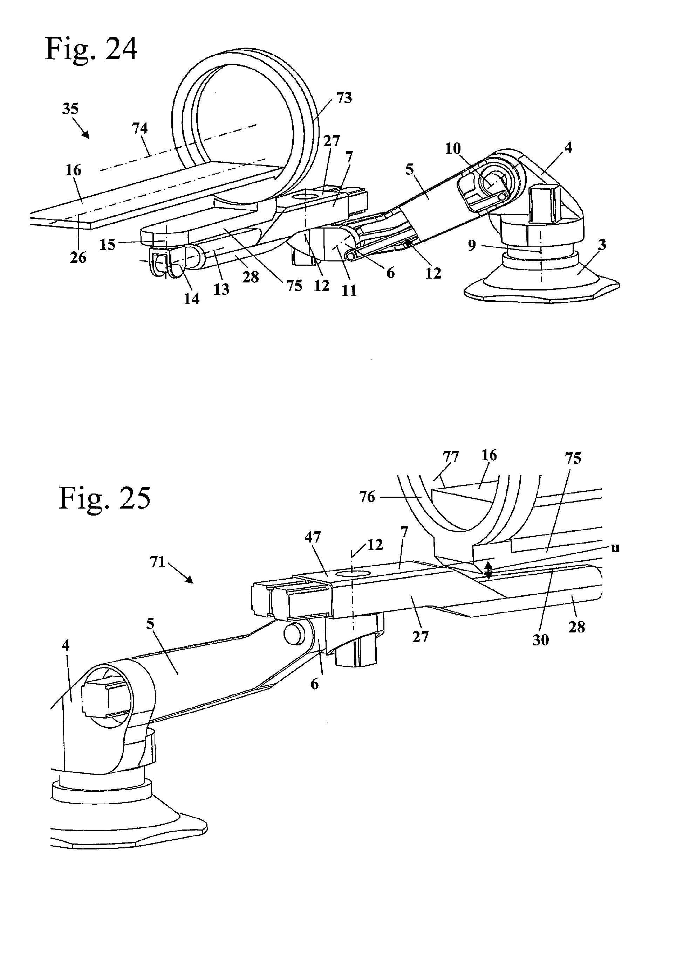

Die Figuren 22 bis 25 zeigen ein Ausführungsbeispiel einer Patientenpositioniereinrichtung 71. Die Patientenpositioniereinrichtung 71 besitzt eine Schwenkeinrichtung 73, über die die Patientenliege 16 am Handabschnitt 8 des Roboterarms 2 gehalten ist. Die Schwenkeinrichtung 73 ist dabei benachbart zu einer Breitseite 48 der Patientenliege 16 angeordnet. Der Handabschnitt 8 ist etwa mittig unter der Patientenliege 16 angeordnet und über einen Träger 75 mit der Schwenkeinrichtung 73 verbunden. Dadurch können die in den Arm 7 eingeleiteten Biegemomente verringert werden. Die Schwenkeinrichtung 73 ermöglicht eine Verschwenkung der Patientenliege 16 um eine Schwenkachse 74, die parallel zur Längsmittelachse 26 der Patientenliege 16 liegt. Fig. 25 zeigt die Anordnung in Bestrahlungsposition 35. In dieser Position ist der Handabschnitt 8 unter der Patientenliege 16 angeordnet. Auch der Träger 75 befindet sich unter der Patientenliege 16.

Fig. 23 zeigt die Anordnung in Aufstiegsposition 36. Die Schwenkeinrichtung 73 hat die Patientenliege 16 um die Schwenkachse 74 um etwa 90° verschwenkt. Der Roboterarm 2 hat den Träger 75 in Gegenrichtung verschwenkt, so dass die horizontale Ausrichtung der Patientenliege 16 erhalten geblieben ist. Der Träger 75 ist ebenso wie der Arm 7 in dieser Aufstiegsposition 36 seitlich neben der Patientenliege 16 und oberhalb der Ebene der Oberseite 33 der Patientenliege 16 angeordnet. Die Schwenkeinrichtung 73 besitzt einen ersten Ring 76, der am Träger 75 fixiert ist, sowie einen zweiten Ring 77, der an der Patientenliege 16 fixiert ist. Der Innendurchmesser der Ringe 76 und 77 ist größer als die Breite der Patientenliege 16. Die minimale Aufstiegshöhe g ist durch den vertikalen Abstand der Unterseite 34 der Patientenliege 16 zur Unterseite der Ringe 76 und 77, also der dem Raumboden 25 zugewandten Seite der Ringe 76 und 77, bei horizontal ausgerichteter Aufnahmefläche 31 vorgegeben. In Aufstiegsposition 36 ist der Handabschnitt 8 oberhalb der Ebene der Aufnahmefläche 31 angeordnet. Wie die Figuren 24 und 25 zeigen, ist der Arm 7 so abgekröpft, dass der Träger 75 über den Arm 7 schwenken kann. Wie Fig. 25 zeigt, besitzt die Oberseite 47 des ersten Abschnitts 27 zur Oberseite 30 des zweiten Abschnitts 28 des Arms 7 einen vertikalen Abstand u. Dadurch kann die Patientenliege 16 um die Rotationsbewegungsachse 15 frei um 360° verschwenkt werden. Gleichzeitig kann aufgrund der Kröpfung des Arms 7 eine niedrige Bestrahlungsposition 35 erreicht werden.

Es kann vorgesehen sein, dass die Patientenliege 16 an der Schwenkeinrichtung 73 horizontal verfahrbar angeordnet ist. An der Patientenliege 16 kann zusätzlich eine bildgebende Einrichtung angeordnet sein. Die bildgebende Einrichtung muss für die Aufstiegsposition 36 nicht zwingend gegenüber dem Handabschnitt 8 verschwenkt werden, sondern kann fest am Handabschnitt 8 angeordnet sein und selbst eine Verschwenkung um eine parallel zur Längsmittelachse 26 liegende Schwenkachse zulassen, so dass die Elemente der bildgebenden Einrichtung beim Aufstieg eines Patienten seitlich weggeschwenkt werden können.

## Patentansprüche

1. Patientenpositioniereinrichtung mit einer Bewegungseinrichtung zur Bewegung einer Patientenaufnahme, wobei die Bewegungseinrichtung Translationsbewegungen der Patientenaufnahme in alle drei Raumrichtungen und Rotationsbewegungen um alle drei Raumachsen (x, y, z) ermöglicht,
**dadurch gekennzeichnet, dass** die Patientenaufnahme mit der Bewegungseinrichtung über eine Schwenkeinrichtung (23, 53, 63, 73) verbunden ist, die eine Schwenkbewegung der Patientenaufnahme gegenüber der Bewegungseinrichtung um eine horizontale Schwenkachse (24, 54, 64, 74) ermöglicht.

2. Patientenpositioniereinrichtung nach Anspruch 1,
**dadurch gekennzeichnet, dass** die Schwenkachse (24, 54, 64, 74) der Schwenkeinrichtung (23, 53, 63, 73) parallel zu einer Längsmittelachse (26) der Patienten-aufnahme verläuft.

3. Patientenpositioniereinrichtung nach Anspruch 1 oder 2,
**dadurch gekennzeichnet, dass** die Schwenkeinrichtung (23, 53, 63, 73) eine Schwenkbewegung der Patientenaufnahme gegenüber der Bewegungseinrichtung um einen Winkel (α) von mindestens 60° um die Schwenkachse (24, 54, 64, 74) der Schwenkrichtung (23, 53, 63, 73) ermöglicht.

4. Patientenpositioniereinrichtung nach einem der Ansprüche 1 bis 3,
**dadurch gekennzeichnet, dass** die Schwenkachse (24) der Schwenkeinrichtung (23) mit einer Längsmittelachse (26) der Patientenaufnahme zusammenfällt.

5. Patientenpositioniereinrichtung nach einem der Ansprüche 1 bis 3,
**dadurch gekennzeichnet, dass** die Patientenaufnahme eine Aufnahmefläche (31) für einen Patienten (32) aufweist, und dass die Patientenaufnahme bei horizontal angeordneter Aufnahmefläche (31) eine dem Raumboden (25) zugewandte Unterseite (34) aufweist, wobei der Abstand (c, i, k) der Schwenkachse (24, 54, 64, 74) zum Raumboden (25) mindestens dem Abstand (b) der Unterseite (34) der Patientenaufnahme zum Raumboden (25) entspricht.

6. Patientenpositioniereinrichtung nach Anspruch 5,
**dadurch gekennzeichnet, dass** die Schwenkachse (54) der Schwenkeinrichtung (53) bei horizontal angeordneter Aufnahmefläche (31) einen horizontalen Abstand (e) zur Längsmittelachse (26) der Patientenaufnahme besitzt.

7. Patientenpositioniereinrichtung nach Anspruch 5,
**dadurch gekennzeichnet, dass** die Schwenkachse (64, 74) der Schwenkeinrichtung (63, 73) bei horizontal angeordneter Aufnahmefläche (31) einen vertikalen Abstand (f) zur Längsmittelachse (26) der Patientenaufnahme besitzt.

8. Patientenpositioniereinrichtung nach einem der Ansprüche 1 bis 7,
**dadurch gekennzeichnet, dass** die Bewegungseinrichtung ein Roboterarm (2) ist.

9. Patientenpositioniereinrichtung nach Anspruch 8,
**dadurch gekennzeichnet, dass** der Roboterarm (2) mindestens sechs Rotationsbewegungsachsen (9, 10, 11, 12, 13, 14, 15) aufweist, wobei der Roboterarm (2) einen Handabschnitt (8) aufweist, der Bewegungen der Patientenaufnahme um alle drei Raumachsen (x, y, z) ermöglicht, und wobei die Schwenkeinrichtung (23, 53, 63, 73) an dem Handabschnitt (8) des Roboterarms (2) festgelegt ist.

10. Patientenpositioniereinrichtung nach Anspruch 9,
**dadurch gekennzeichnet, dass** die Patientenpositioniereinrichtung (1, 51, 61, 71) eine Bestrahlungsposition (35) aufweist, in der der Handabschnitt (8) zum Raumboden (25) einen Abstand (1) besitzt, der kleiner als ein Abstand (b) der Unterseite (34) der Patientenaufnahme zum Raumboden (25) ist, und dass die Patienten-positioniereinrichtung (1, 51, 61, 71) eine Aufstiegsposition (36) besitzt, in der der Abstand (m) des Handabschnitts (8) zum Raumboden (25) größer ist als der Abstand (n) der Unterseite (34) der Patientenaufnahme zum Raumboden (25).

11. Patientenpositioniereinrichtung nach einem der Ansprüche 8 bis 10,
**dadurch gekennzeichnet, dass** der Roboterarm (2) ein Grundgestell (3) besitzt, an dem ein Karussell (4) um eine erste Rotationsbewegungsachse (9) schwenkbar gelagert ist, und dass der Roboterarm (2) eine Schwinge (5) besitzt, die gegenüber dem Karussell (4) um eine zweite Rotationsbewegungsachse (10) schwenkbar gelagert ist.

12. Patientenpositioniereinrichtung nach Anspruch 11,
**dadurch gekennzeichnet, dass** der Roboterarm (2) einen Arm (7) aufweist, der gegenüber der Schwinge (5) um eine dritte, horizontal angeordnete Rotationsbewegungsachse (11) schwenkbar gelagert ist.

13. Patientenpositioniereinrichtung nach Anspruch 12,
**dadurch gekennzeichnet, dass** der Arm (7) an der Schwinge (5) über einen Zwischenabschnitt (6) festgelegt ist, wobei der Zwischenabschnitt (6) an der Schwinge (5) um die dritte Rotationsbewegungsachse (11) schwenkbar gelagert ist, wobei die zweite Rotationsbewegungsachse (10) und die dritte Rotationsbewegungsachse (11) parallel zueinander verlaufen und wobei die Rotationsbewegung des Zwischenabschnitts (6) um die dritte Rotationsbewegungsachse (11) an die Bewegung der Schwinge (5) um die zweite Rotationsbewegungsachse (10) über eine Koppeleinrichtung (19) gekoppelt ist.

14. Patientenpositioniereinrichtung nach Anspruch 13,
**dadurch gekennzeichnet, dass** die Koppeleinrichtung (19) ein Koppelelement umfasst, das in einem Abstand (o) zur zweiten Rotationsbewegungsachse (10) um eine erste Lagerachse (21) schwenkbar an dem Karussell (4) gehalten ist und das in einem Abstand (p) zur dritten Rotationsbewegungssachse (11) um eine zweite Lagerachse (22) schwenkbar an dem Zwischenelement (6) gehalten ist.

15. Patientenpositioniereinrichtung nach einem der Ansprüche 12 bis 14,
**dadurch gekennzeichnet, dass** der Arm (7) gekröpft ausgebildet ist, wobei der Arm (7) einen ersten Abschnitt (27) besitzt, mit dem der Arm (7) an dem Zwischenelement (6) gehalten ist, und wobei der Arm (7) einen zweiten Abschnitt (28) besitzt, an dem der Handabschnitt (8) angeordnet ist, wobei der Abstand (q) der Oberseite (47) des ersten Abschnitts (27) zum Raumboden (25) größer als der Abstand (r) der Oberseite (30) des zweiten Abschnitts (28) zum Raumboden (25) ist.
